# EUROPEAN PATENT APPLICATION

(11) **EP 3 932 450 A1**
(43) Date of publication of application: **05.01.2022**
(21) Application number: 20784086.9
(22) Date of filing: 04.03.2020
(51) Int. Cl.: A61M 5/142, A61M 5/145, A61M 5/168

(54) **LIQUID MEDICINE ADMINISTRATION APPARATUS**

(30) Priority: 29.03.2019 JP 2019066300
(71) Applicant: TERUMO Kabushiki Kaisha, Tokyo 151-0072 (JP)
(72) Inventor: FURUICHI,Yoko, Ashigarakami-gun, Kanagawa 259-0151 (JP); YAKUSHIJI,Yusuke, Ashigarakami-gun, Kanagawa 259-0151 (JP)
(74) Representative: Casalonga
(86) International application number: PCT/JP2020/009151
(87) International publication number: WO 2020/203004

(57) **Abstract**

Provided is a liquid medicine administration device capable of detecting an abnormality in administration of a liquid medicine.

A liquid medicine administration device 100 includes: a plunger 130 that pushes a liquid medicine in a liquid medicine container 110 filled with the liquid medicine; a supply path that causes a distal end opening of the liquid medicine container 110 to communicate with a needle in order to administer the liquid medicine from the needle pierced into a living body; a drive mechanism 140 that advances the plunger 130 toward the distal end opening of the liquid medicine container 110 in order to discharge the liquid medicine from the liquid medicine container 110; and a control unit 160 that controls operation of the drive mechanism 140, in which the drive mechanism 140 includes a DC motor 141 that applies, to the plunger 130, a drive force for advancing the plunger 130, and a rotation detection unit 143 that detects a rotation of the DC motor 141, and the control unit 160 has a rotation speed calculation function of calculating a rotation speed of the DC motor 141 based on the rotation of the DC motor 141 detected by the rotation detection unit 143, and detects an abnormality in administration when the rotation speed of the DC motor 141 calculated by the rotation speed calculation function is lower than a predetermined lower limit rotation speed.

## Description

### Technical Field

The present invention relates to a liquid medicine administration device capable of detecting an abnormality in administration of a liquid medicine.

### Background Art

In the related art, as disclosed in Patent Literature 1, there is known a syringe pump type liquid medicine administration device that administers a liquid medicine filled in a liquid medicine container to a living body. The syringe pump type liquid medicine administration device includes a drive mechanism and a control unit, and continuously administers a liquid medicine with high accuracy for a long time by moving a plunger little by little by the drive mechanism.

### Citation List

### Patent Literature

Patent Literature 1: JP 2015-181869 A

### Summary of Invention

### Technical Problem

At the time of using the liquid medicine administration device, when a power switch is turned on, the control unit drives the drive mechanism, and moves the plunger little by little to administer the liquid medicine to the living body. However, in a case where a supply path for administering the liquid medicine to the living body is blocked for some reason, the liquid medicine leaks from the middle of the supply path since pressure of the plunger cannot be withstood, and the liquid medicine administration device itself is damaged since the drive mechanism or the motor is overloaded. In a case where the liquid medicine has leaked out, the load on the motor decreases, and thus the abnormality in administration cannot be detected even when it is detected whether or not the motor is rotating.

An object of the present invention is to provide a liquid medicine administration device capable of detecting an abnormality in administration of a liquid medicine.

### Solution to Problem

In order to achieve the object described above, an aspect of the present invention provides a liquid medicine administration device including: a plunger that pushes a liquid medicine in a liquid medicine container filled with the liquid medicine; a supply path that causes a distal end opening of the liquid medicine container to communicate with a needle in order to administer the liquid medicine from the needle pierced into a living body; a drive mechanism that advances the plunger toward the distal end opening of the liquid medicine container in order to discharge the liquid medicine from the liquid medicine container; and a control unit that controls operation of the drive mechanism, in which the drive mechanism includes a DC motor that applies, to the plunger, a drive force for advancing the plunger, and a rotation detection unit that detects a rotation of the DC motor, and the control unit has a rotation speed calculation function of calculating a rotation speed of the DC motor based on the rotation of the DC motor detected by the rotation detection unit, and detects an abnormality in administration when the rotation speed of the DC motor calculated by the rotation speed calculation function is lower than a predetermined lower limit rotation speed.

In order to achieve the object described above, another aspect of the present invention provides a liquid medicine administration device including: a plunger that pushes a liquid medicine in a liquid medicine container filled with the liquid medicine; a supply path that causes a distal end opening of the liquid medicine container to communicate with a needle in order to administer the liquid medicine from the needle pierced into a living body; a drive mechanism that advances the plunger toward the distal end opening of the liquid medicine container in order to discharge the liquid medicine from the liquid medicine container; and a control unit that controls operation of the drive mechanism, in which the drive mechanism includes a DC motor that applies, to the plunger, a drive force for advancing the plunger, and the control unit has a current value measurement function of measuring a value of a motor current flowing in the DC motor, and detects an abnormality in administration when the value of the motor current exceeds a predetermined upper limit current value.

In order to achieve the object described above, still another aspect of the present invention provides a liquid medicine administration device including: a plunger that pushes a liquid medicine in a liquid medicine container filled with the liquid medicine; a supply path that causes a distal end opening of the liquid medicine container to communicate with a needle in order to administer the liquid medicine from the needle pierced into a living body; a drive mechanism that advances the plunger toward the distal end opening of the liquid medicine container in order to discharge the liquid medicine from the liquid medicine container and includes a DC motor that applies, to the plunger, a drive force for advancing the plunger; a control unit that controls operation of the drive mechanism; and a current limiting circuit that limits a current so that any current equal to or greater than a predetermined current does not flow in the DC motor.

### Advantageous Effects of Invention

According to the aspects of the present invention, in the liquid medicine administration device, since the abnormality in administration of the liquid medicine can be detected, it is possible to prevent the liquid medicine from leaking out or the liquid medicine administration device from being damaged.

### Brief Description of Drawings

Fig. 1 is a side view of a liquid medicine administration system.
Fig. 2 is a view schematically illustrating a usage example of a liquid medicine administration system.
Fig. 3 is a schematic perspective view of a liquid medicine administration device.
Fig. 4 is a schematic perspective view of a chassis included in a housing and each component member assembled to the chassis.
Fig. 5 is a plan view of a liquid medicine administration device illustrating a state before a plunger is moved forward.
Fig. 6 is a plan view of a liquid medicine administration device illustrating a state after the plunger is moved forward.
Fig. 7 is a block diagram of a control system of a liquid medicine administration device according to a first embodiment.
Fig. 8 is a characteristic diagram of a DC motor.
Fig. 9 is an operation flowchart of a control unit according to the first embodiment.
Fig. 10 is a block diagram of a control system of a liquid medicine administration device according to a second embodiment.
Fig. 11 is an operation flowchart of a control unit according to the second embodiment.
Fig. 12 is a block diagram of a control system of a liquid medicine administration device according to a third embodiment.
Fig. 13 is a diagram illustrating an example of a current limiting circuit of Fig. 12.
Fig. 14 is an operation flowchart of a control unit according to a third embodiment.

### Description of Embodiments

Hereinafter, embodiments of the present invention will be described with reference to the accompanying drawings. Note that, the following description does not limit the technical scope or meaning of terms described in the claims. Furthermore, dimensional ratios in the drawings are exaggerated for convenience of description, and may be different from actual ratios.

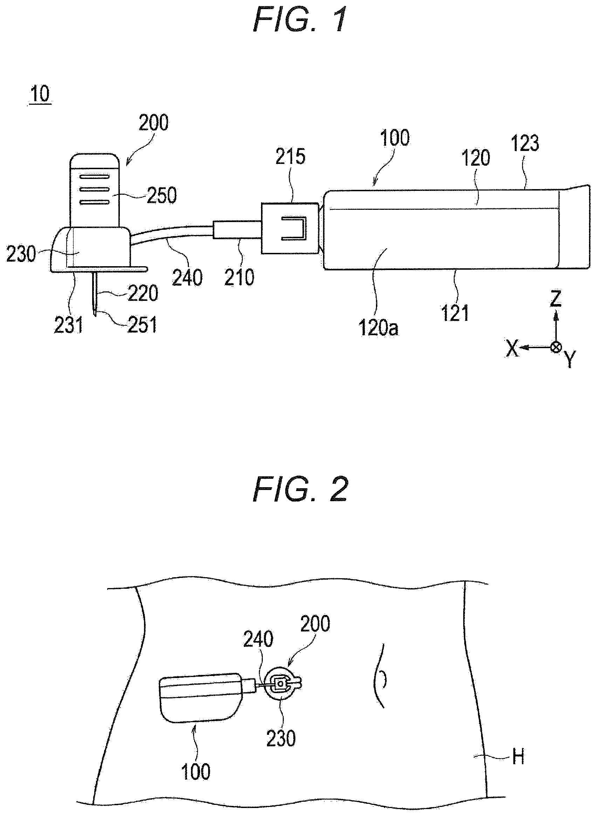

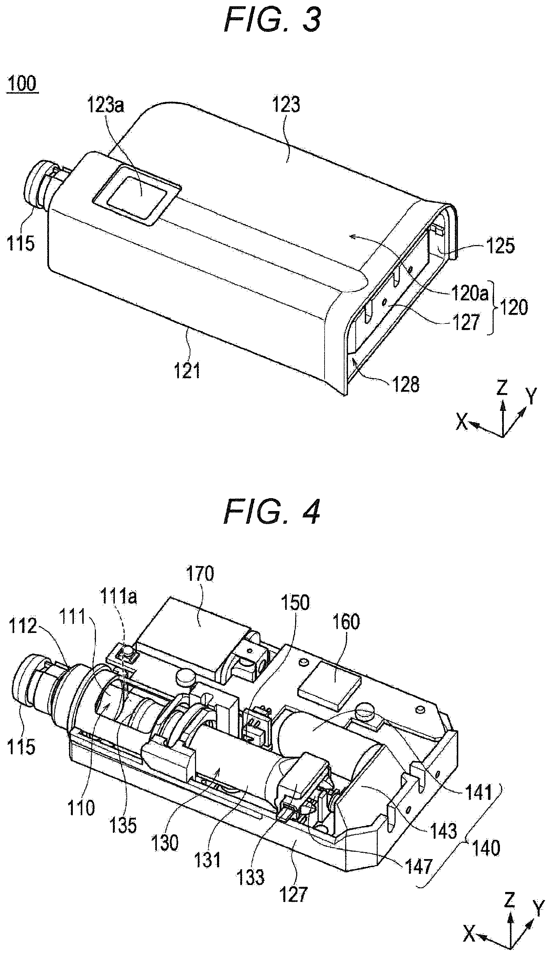

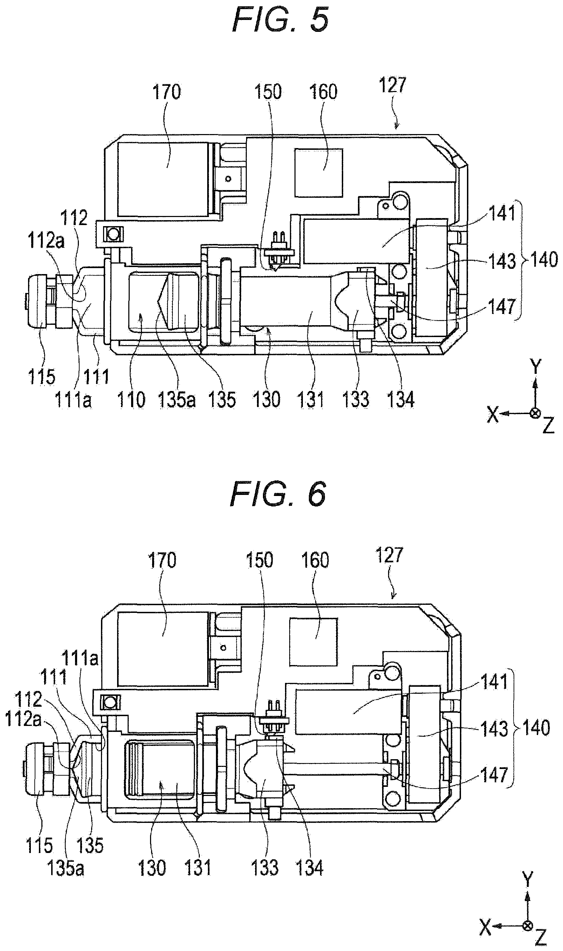

Figs. 1 to 6 are views for explaining a liquid medicine administration system 10, a liquid medicine administration device 100, and an administration tool 200 according to the present embodiment. Fig. 1 is a side view of a liquid medicine administration system. Fig. 2 is a view schematically illustrating a usage example of the liquid medicine administration system. Fig. 3 is a schematic perspective view of a liquid medicine administration device. Fig. 4 is a schematic perspective view of a chassis included in a housing and each component member assembled to the chassis. Fig. 5 is a plan view of the liquid medicine administration device illustrating a state before a plunger is moved forward. Fig. 6 is a plan view of the liquid medicine administration device illustrating a state after the plunger is moved forward. Note that, an arrow X in each drawing indicates a "longitudinal direction (longitudinal direction of a liquid medicine container 110)" of the liquid medicine administration device 100, an arrow Y indicates a "width direction (depth direction)" of the liquid medicine administration device 100, and an arrow Z indicates a "height direction" of the liquid medicine administration device 100.

### (Liquid Medicine Administration System)

The liquid medicine administration system 10 is used to administer a liquid medicine into a living body. As illustrated in Fig. 1, the liquid medicine administration system 10 includes the liquid medicine administration device 100 and the administration tool 200.

As illustrated in Fig. 2, the liquid medicine administration device 100 and the administration tool 200 are configured as a patch type used by being stuck on a body surface (skin) H of a user. A body part of the user to which the liquid medicine administration device 100 and the administration tool 200 are attached is not particularly limited, but is, for example, an abdomen or a femoral part.

For example, the liquid medicine administration system 10 can continuously administer a liquid medicine (not illustrated) filled in the liquid medicine container 110 included in the liquid medicine administration device 100 into the living body for a relatively long time (for example, approximately several minutes to several hours) by a pressing action of a plunger 130 (see Fig. 4) to be described later. Note that, the liquid medicine administration system 10 may intermittently administer the liquid medicine into the living body.

### (Liquid Medicine Administration Device)

As illustrated in Figs. 3 to 6, the liquid medicine administration device 100 includes the liquid medicine container 110 including a cylindrical (barrel-shaped) main body 111 filled with the liquid medicine, a housing 120 that holds the liquid medicine container 110, the plunger 130 that pushes the liquid medicine in the liquid medicine container 110, a drive mechanism 140 that advances the plunger 130 toward a distal end opening of the liquid medicine container 110, a detection unit 150 that detects a portion 134 to be detected of the plunger 130 and detects completion of feeding of the liquid medicine based on a detection result, and a control unit 160 that controls operation of the drive mechanism 140.

As illustrated in Figs. 3 and 4, the housing 120 includes a box-shaped housing main body 120a in which an accommodation space 128 is formed, and a chassis (corresponding to a "support portion") 127 which is accommodated in the accommodation space 128 of the housing main body 120a and can be fixed to the housing main body 120a.

As illustrated in Fig. 3, a window portion 123a that allows the inside of the accommodation space 128 to be visually recognized from the outside of the housing 120 is formed on an upper surface 123 of the housing main body 120a. The window portion 123a is formed by providing a transparent or translucent portion in a part of the housing main body 120a.

A proximal end opening 125 for inserting a chassis 127 into the accommodation space 128 of the housing main body 120a is formed on a proximal end side in a longitudinal direction of the housing main body 120a. The proximal end opening 125 of the housing main body 120a is closed by a lid member (not illustrated) in a state in which the chassis 127 is accommodated in the accommodation space 128.

A bottom surface 121 of the housing main body 120a is provided with a sheet-like sticking portion (not illustrated) that can be stuck to the body surface H of the user. In an initial state before the liquid medicine administration device 100 is attached to the user, a peelable protective sheet is attached to a sticking surface of the sticking portion.

As illustrated in Fig. 4, the chassis 127 holds the liquid medicine container 110, the plunger 130, the drive mechanism 140, the detection unit 150, the control unit 160, and a power supply unit 170.

The liquid medicine container 110 is a so-called prefilled liquid medicine container. Therefore, the liquid medicine is filled in a lumen 111a of the main body 111 of the liquid medicine container 110 in advance. Examples of the liquid medicine include protein preparations, narcotic analgesics, diuretics, and the like.

A sealing member (not illustrated) for preventing leakage of the liquid medicine is disposed in the distal end opening (discharge port) formed at a distal end 112 of the liquid medicine container 110. As illustrated in Fig. 3, the distal end opening of the liquid medicine container 110 is disposed so as to protrude outward from the housing main body 120a. Furthermore, an attachment portion 115 that is connected to a tube 240 (see Fig. 1) to be described later is attached to a distal end portion of the liquid medicine container 110, the distal end portion protruding from the housing main body 120a.

A main body 131 of the plunger 130 is inserted into the lumen 111a of the main body 111 of the liquid medicine container 110 (see Figs. 4 and 5). A gasket 135 slidable on an inner wall of the liquid medicine container 110 is disposed at a distal end of the main body 131 of the plunger 130. The gasket 135 liquid-tightly seals a proximal end side of the gasket 135 by liquid-tightly bringing an outer circumferential portion of the gasket 135 into close contact with an inner circumferential surface of the main body 111 of the liquid medicine container 110.

In the present embodiment, the gasket 135 is configured to be shrinkable in a direction (longitudinal direction) in which the plunger 130 advances when the plunger 130 advances in a state in which the gasket 135 abuts against a distal end inner wall 112a (see Fig. 5) of the liquid medicine container 110. The gasket 135 can be made of, for example, a flexible resin material such as a rubber material or an elastomer so as to be shrinkable as described above.

As illustrated in Fig. 5, the gasket 135 has a tapered shape in which an outer diameter decreases toward a distal end side. Furthermore, the shape of the gasket 135 is substantially the same as the shape of the distal end inner wall 112a of the liquid medicine container 110.

As illustrated in Fig. 5, the portion 134 to be detected is provided at a proximal end of the plunger 130. The portion 134 to be detected is used to detect completion of feeding of the liquid medicine by the liquid medicine administration device 100.

The control unit 160 controls a liquid medicine feeding operation of the liquid medicine administration device 100. The control unit 160 can be configured by, for example, a known microcomputer (electronic circuit element) on which a CPU, a RAM, a ROM, and the like are mounted. The control unit 160 integrally controls operations of the drive mechanism 140, the detection unit 150, and the power supply unit 170.

As illustrated in Fig. 5, the detection unit 150 is disposed in the chassis 127. As illustrated in Fig. 6, the detection unit 150 detects completion of feeding of the liquid medicine of the liquid medicine administration device 100 when the portion 134 to be detected included in the plunger 130 comes into contact with the detection unit 150. The detection unit 150 can be configured by, for example, a known contact-type sensor that transmits a predetermined electric signal when the portion 134 to be detected comes into contact with the detection unit 150. The control unit 160 acquires information regarding completion of feeding of the liquid medicine by receiving the electric signal from the portion 134 to be detected. Note that, when the plunger 130 advances by a predetermined amount, the specific configuration and the like of the detection unit 150 are not particularly limited as long as a position of the portion 134 to be detected of the plunger 130 can be detected.

The power supply unit 170 can be configured by, for example, a known button battery or the like. The liquid medicine administration device 100 is required to be downsized. Therefore, a small button battery is used as the power supply unit 170.

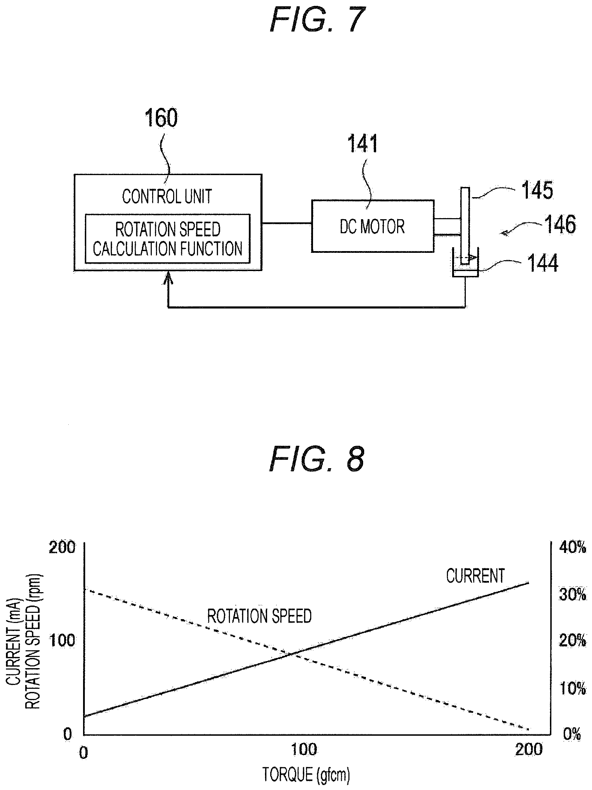

As illustrated in Fig. 4, the drive mechanism 140 includes a DC motor 141 that receives a drive current from the power supply unit 170 and applies a drive force, a speed reduction mechanism 143 that includes a gear or the like transmitting the drive force of the DC motor 141, an encoder (see Fig. 7) that is provided adjacent to the speed reduction mechanism 143 and includes a photointerrupter as a rotation detection unit that detects a rotation of the DC motor 141 and a slit plate that rotates in accordance with the rotation of the DC motor 141, and a feed screw 147 that is connected to the speed reduction mechanism 143.

The feed screw 147 is connected to a proximal end connection portion 133 disposed in the vicinity of the proximal end of the plunger 130. The feed screw 147 converts a rotational motion transmitted from the speed reduction mechanism 143 into a linear motion to advance the plunger 130 in the longitudinal direction (X direction). The plunger 130 advances toward a distal end side of the liquid medicine container 110 to push the liquid medicine from the lumen 111a of the main body 111 of the liquid medicine container 110 to the tube 240 (see Fig. 1).

### (Administration Tool)

As illustrated in Figs. 1 and 2, the administration tool 200 is configured to be connectable to the liquid medicine administration device 100.

The administration tool 200 includes a connector 210, a needle tube 220 that punctures the living body, a puncture unit (cannula housing) 230, the tube 240, and a puncture assisting tool 250 that assists in puncturing the living body with the needle tube 220.

The connector 210 is configured to be connectable to the liquid medicine administration device 100 via an attachment portion 215 fixed to the connector 210. The attachment portion 215 can be connected to the liquid medicine administration device 100 by being externally fitted to the attachment portion 115 (see Fig. 4) provided in the vicinity of the distal end 112 of the liquid medicine container 110 protruding to the outside of the housing 120.

Inside the attachment portion 215, a connection needle portion (not illustrated) through which the sealing member (not illustrated) disposed at a distal end portion of the liquid medicine container 110 can be inserted is disposed. The tube 240 communicates with the lumen 111a of the main body 111 of the liquid medicine container 110 via the connection needle portion.

Inside the puncture unit 230, a flow path (not illustrated) through which the tube 240 communicates with a lumen of the needle tube 220 is formed. The liquid medicine fed to the puncture unit 230 through the tube 240 is administered into the living body through the flow path formed inside the puncture unit 230 and the needle tube 220.

When the liquid medicine is fed to the user, the puncture assisting tool 250 is attached to the puncture unit 230. The puncture assisting tool 250 holds an introduction needle (inner needle) 251. The introduction needle 251 protrudes from a distal end of the needle tube 220 in a state in which the puncture assisting tool 250 is attached to the puncture unit 230. By puncturing the living body with the needle tube 220 in a state in which the introduction needle 251 is inserted into the needle tube 220, the user can insert the needle tube 220 into the living body while preventing the needle tube 220 from being broken or the like.

The puncture assisting tool 250 is removed from the puncture unit 230 after puncturing the living body with the needle tube 220. When the puncture assisting tool 250 is removed from the puncture unit 230, the introduction needle 251 is removed from the lumen of the needle tube 220.

After puncturing the living body with the needle tube 220, the puncture assisting tool 250 is removed, and the puncture unit 230 is left on the body surface H of the user in a state in which the needle tube 220 is indwelled in the living body. When the plunger 130 of the liquid medicine administration device 100 advances in the liquid medicine container 110 in this state, the liquid medicine filled in the liquid medicine container 110 is fed to the lumen of the needle tube 220 via the tube 240 and the flow path of the puncture unit 230.

The introduction needle 251 can be formed of, for example, a metal needle. Furthermore, the needle tube 220 can be formed of, for example, a resin tubular member (cannula).

Similarly to the liquid medicine administration device 100, the administration tool 200 is configured as a patch type used by being stuck on the body surface H of the user. A sheet-like sticking portion (not illustrated) that can be stuck to the body surface H is provided on a contact surface (bottom surface) 231 of the puncture unit 230 of the administration tool 200. In an initial state before the administration tool 200 is attached to the user, a peelable protective sheet is attached to a sticking surface of the sticking portion.

As described above, a schematic configuration of the liquid medicine administration system 10, the liquid medicine administration device 100, and the administration tool 200 has been described. The liquid medicine administration device 100 is required to be reduced in size and cost in order to facilitate handling at the time of use and to save a storage space at the time of storage. Therefore, as the DC motor 141, a coreless DC motor, which is easily downsized and has high torque efficiency with respect to electric power, is used. The DC motor has a characteristic that a current supplied to the DC motor and a rotation speed of the DC motor are different depending on the magnitude of a load torque. The control unit 160 detects the abnormality in administration by using the characteristic of the DC motor 141. Therefore, the control unit 160 controls the drive mechanism 140 as follows. Control of the drive mechanism 140 by the control unit 160 will be described separately in the first embodiment to the third embodiment.

### [First Embodiment]

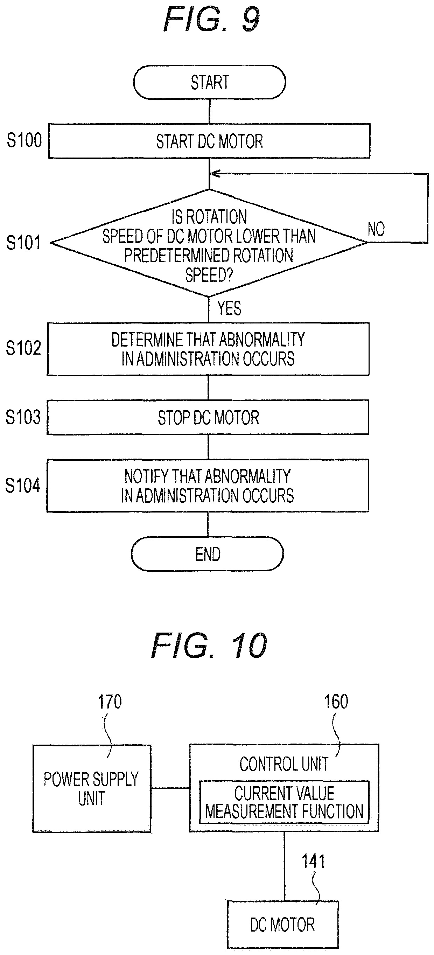

A specific operation of the control unit 160 according to the first embodiment will be described with reference to Figs. 7 to 9. Fig. 7 is a block diagram of a control system of the liquid medicine administration device 100 according to the first embodiment. Fig. 8 is a characteristic diagram of the DC motor 141. Fig. 9 is an operation flowchart of the control unit 160 according to the first embodiment.

The control unit 160 is electrically connected to the DC motor 141. A rotation shaft of the DC motor 141 is mechanically connected to the speed reduction mechanism 143. An encoder 146 as a rotation detection unit that detects a rotation of the DC motor 141 is provided adjacent to the speed reduction mechanism 143. The encoder 146 includes a photointerrupter 144 including an optical sensor and a slit plate 145 in which a large number of slits are radially formed, and detects the rotation of the DC motor 141 by detecting whether or not light passes through the slits of the slit plate 145 with the optical sensor of the photointerrupter 144. The photointerrupter 144 is electrically connected to the control unit 160. The control unit 160 has a rotation speed calculation function of calculating the rotation speed of the DC motor 141 based on the rotation of the DC motor 141 detected by the encoder 146. Furthermore, the control unit 160 detects the abnormality in administration when the rotation speed of the DC motor 141 calculated by the rotation speed calculation function is lower than a predetermined lower limit rotation speed. Note that, in the present embodiment, the encoder 146 using the photointerrupter 144 as the rotation detection unit has been exemplified, but an encoder using a magnetic sensor may be used.

When the control unit 160 rotates the DC motor 141, the speed reduction mechanism 143 is driven, and the plunger 130 advances in the liquid medicine container 110 (see Figs. 5 and 6). The encoder 146 provided adjacent to the speed reduction mechanism 143 detects the rotation of the DC motor 141, and the control unit 160 calculates the rotation speed of the DC motor 141 based on the rotation of the DC motor 141 detected by the encoder 146. The rotation of the DC motor 141 detected by the encoder 146 is fed back to the control unit 160, and the control unit 160 rotates the DC motor 141 at a preset constant rotation speed in accordance with the feedback According to this, the liquid medicine filled in the liquid medicine container 110 is fed to the lumen of the needle tube 220 via the flow path of the tube 240 and the puncture unit 230, and the liquid medicine is administered to the living body at a constant speed (see Fig. 1).

In characteristics of the DC motor 141 used in the present embodiment, as illustrated in Fig. 8, the current flowing in the DC motor 141 increases as the load increases (torque increases), and conversely, the rotation speed of the DC motor 141 decreases as the load increases.

Therefore, as illustrated in the operation flowchart of Fig. 9, the control unit 160 starts the DC motor 141 when the liquid medicine is administered to the living body (S100), and determines whether or not the rotation speed of the DC motor 141 is lower than a predetermined rotation speed (S101). The rotation speed of the DC motor 141 when the liquid medicine is administered to the living body is set in advance in accordance with the administration speed of the liquid medicine. When the rotation speed of the DC motor 141 is not lower than the predetermined rotation speed (when the rotation speed is not lower than a lower limit rotation speed) (S101: NO), it can be determined that the liquid medicine is normally administered, and thus the liquid medicine is continued to be administered as it is. On the other hand, when the rotation speed of the DC motor 141 is lower than the predetermined rotation speed (when the rotation speed is lower than the lower limit rotation speed), the load on the DC motor 141 increases and it is considered that an abnormality such as blockage of the supply path of the liquid medicine occurs (S101: YES). Therefore, the control unit 160 determines that the abnormality in administration occurs (S102). Next, the control unit 160 stops the DC motor 141 (S103) and notifies a user of the abnormality in administration (S104). In notification of the abnormality in administration, for example, an LED may be provided in a casing of the liquid medicine administration device 100 and the LED may be turned on and blinked, or a speaker may be provided in the casing of the liquid medicine administration device 100 and the speaker may sound. Furthermore, the occurrence of the abnormality in administration may be wirelessly notified to an external computer.

The liquid medicine administration device 100 originally includes the encoder 146 in order to control the rotation speed of the DC motor 141. Therefore, in order to cause the control unit 160 to implement the operation as in the present embodiment, it is only necessary to rewrite a program included in the control unit 160, and thus it is not necessary to change the mechanical configuration of the liquid medicine administration device 100 of the related art. Therefore, the present invention can be inexpensively applied to the liquid medicine administration device 100 of the related art.

### [Second Embodiment]

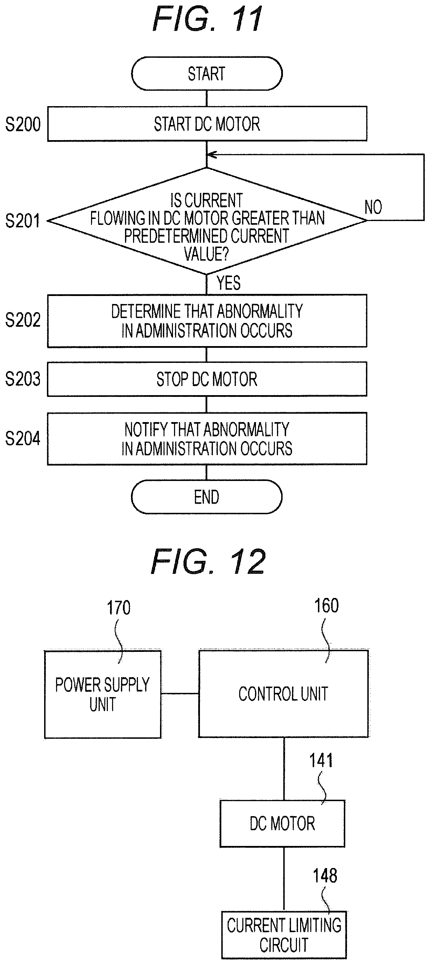

Next, a specific operation of the control unit 160 according to a second embodiment will be described with reference to Figs. 10 and 11. Fig. 10 is a block diagram of a control system of the liquid medicine administration device according to the second embodiment. Fig. 11 is an operation flowchart of the control unit according to the second embodiment.

The control unit 160 is electrically connected to the power supply unit 170. The control unit 160 is electrically connected to the DC motor 141. The DC motor 141 is rotated by electric power supplied from the power supply unit 170. The rotation speed of the DC motor 141 is controlled by the control unit 160. The control unit 160 has a current value measurement function of measuring a value of a motor current flowing in the DC motor 141. Furthermore, the control unit 160 detects the abnormality in administration when the value of the motor current exceeds a predetermined upper limit current value.

When the control unit 160 rotates the DC motor 141, the speed reduction mechanism 143 is driven, and the plunger 130 advances in the liquid medicine container 110 (see Figs. 5 and 6). The control unit 160 rotates the DC motor 141 at a preset constant rotation speed. According to this, the liquid medicine filled in the liquid medicine container 110 is fed to the lumen of the needle tube 220 via the flow path of the tube 240 and the puncture unit 230, and the liquid medicine is administered to the living body at a constant speed (see Fig. 1).

In characteristics of the DC motor 141 used in the present embodiment, as illustrated in Fig. 8, the current flowing in the DC motor 141 increases as the load increases (torque increases), and conversely, the rotation speed of the DC motor 141 decreases as the load increases.

Therefore, as illustrated in the operation flowchart of Fig. 11, the control unit 160 starts the DC motor 141 when the liquid medicine is administered to the living body

(S200), and determines whether or not the current flowing in the DC motor 141 is greater than a predetermined current value (S201). When the current flowing in the DC motor 141 is not greater than the predetermined current value (when the current does not exceed an upper limit current value) (S201: NO), it can be determined that the liquid medicine is normally administered, and thus the liquid medicine is continued to be administered as it is. On the other hand, when the current flowing in the DC motor 141 is greater than the predetermined current value (current exceeds the upper limit current value), the load on the DC motor 141 increases and it is considered that an abnormality such as blockage of the supply path of the liquid medicine occurs (S201: YES). Therefore, the control unit 160 determines that the abnormality in administration occurs (S202).

Next, the control unit 160 stops the DC motor 141 (S203) and notifies a user of the abnormality in administration (S204). A form of the notification of the abnormality in administration is the same as that in the first embodiment.

### [Third Embodiment]

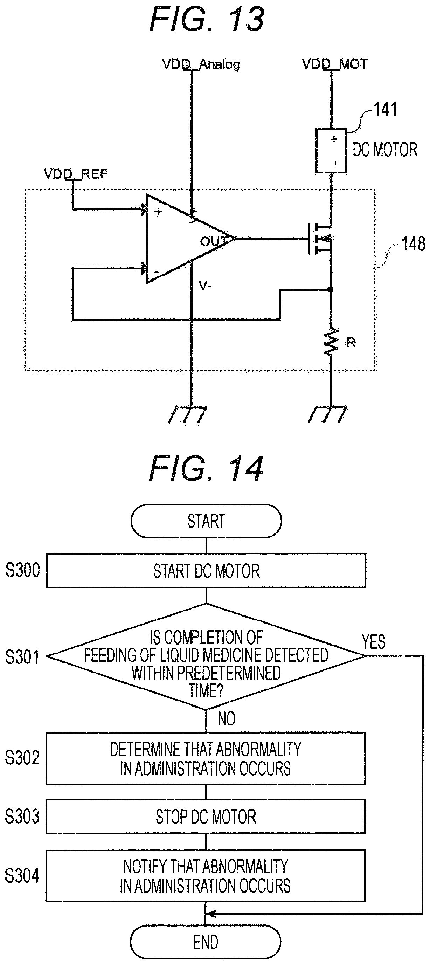

Next, a specific operation of the control unit 160 according to a third embodiment will be described with reference to Figs. 12 to 14. Fig. 12 is a block diagram of a control system of the liquid medicine administration device according to the third embodiment. Fig. 13 is a diagram illustrating an example of a current limiting circuit of Fig. 12. Fig. 14 is an operation flowchart of the control unit according to the third embodiment.

The control unit 160 is electrically connected to the power supply unit 170. The control unit 160 is electrically connected to the DC motor 141. The DC motor 141 is connected to a current limiting circuit 148. The DC motor 141 is rotated by electric power supplied from the power supply unit 170. The rotation speed of the DC motor 141 is controlled by the control unit 160. The current limiting circuit 148 limits the current so that any current equal to or greater than a predetermined current does not flow in the DC motor 141. The current limiting circuit 148 can be realized as a circuit as illustrated in Fig. 13 as an example. Since the circuit of Fig. 13 is a general known circuit, it will not be described in detail.

In characteristics of the DC motor 141 used in the present embodiment, as illustrated in Fig. 8, the current flowing in the DC motor 141 increases as the load increases (torque increases), and conversely, the rotation speed of the DC motor 141 decreases as the load increases. Therefore, when the DC motor 141 rotates at a preset constant rotation speed, the current flowing in the DC motor 141 is not limited to the current limiting circuit 148. On the other hand, when a load is applied to the DC motor 141 and the rotation speed is lower than a preset constant rotation speed, the current flowing in the DC motor 141 increases in accordance with the magnitude of the load. Therefore, the current flowing in the DC motor 141 is limited by the current limiting circuit 148. In a case where the current is limited when the load increases, the DC motor 141 stops due to insufficient torque.

When the control unit 160 rotates the DC motor 141, the speed reduction mechanism 143 is driven, and the plunger 130 advances in the liquid medicine container 110 (see Figs. 5 and 6). The control unit 160 rotates the DC motor 141 at a preset constant rotation speed. When the DC motor 141 rotates at a constant speed, the current flowing in the DC motor 141 is not limited by the current limiting circuit 148. Therefore, the liquid medicine filled in the liquid medicine container 110 is fed to the lumen of the needle tube 220 via the tube 240 and the flow path of the puncture unit 230, and the liquid medicine is administered to the living body at a constant speed (see Fig. 1). On the other hand, in a case where the supply path for administering the liquid medicine to the living body is blocked for some reason, the load on the DC motor 141 increases, the current flowing in the DC motor 141 increases, and the current flowing in the DC motor 141 is limited by the current limiting circuit 148. Therefore, since the load on the DC motor 141 becomes too large, the rotation of the DC motor 141 is stopped. In the present embodiment, the stop of the rotation of the DC motor 141 is detected as follows.

As a first method of detecting that the rotation of the DC motor 141 has stopped, as in the flowchart of Fig. 9 of the first embodiment, there is a method of determining that there is the abnormality in administration when the rotation speed of the DC motor 141 is lower than a predetermined rotation speed (when the rotation speed is lower than the lower limit rotation speed). This method is as described in the first embodiment.

As a second method of detecting that the rotation of the DC motor 141 has stopped, as in the operation flowchart illustrated in Fig. 14, there is a method of determining that there is the abnormality in administration when the detection unit 150 (see Fig. 5 and Fig. 6) does not detect completion of feeding of the liquid medicine of the liquid medicine administration device 100 after a predetermined time has elapsed.

As illustrated in the operation flowchart of Fig. 14, the control unit 160 starts the DC motor 141 when the liquid medicine is administered to the living body (S300). Next, the control unit 160 determines whether or not completion of feeding of the liquid medicine has been detected within a predetermined time (S301). Normally, until a predetermined time elapses after the DC motor 141 is started, the plunger 130 pushes the liquid medicine in the liquid medicine container 110, and the portion 134 to be detected included in the plunger 130 comes into contact with the detection unit 150. However, when the supply path is blocked for some reason, the current limiting circuit 148 acts to extremely slow or stop the rotation of the DC motor 141. Therefore, the portion 134 to be detected included in the plunger 130 does not come into contact with the detection unit 150 until a predetermined time elapses. Therefore, in Step S301, it is determined whether or not the portion 134 to be detected included in the plunger 130 comes into contact with the detection unit 150 within a predetermined time after the control unit 160 starts the DC motor 141. When completion of feeding of the liquid medicine is detected within the predetermined time (S301: YES), since the feeding of the liquid medicine has been normally performed, the processing is ended. On the other hand, when the completion of feeding of the liquid medicine is not detected within the predetermined time, the load on the DC motor 141 increases and it is considered that an abnormality such as blockage of the supply path of the liquid medicine occurs (S301: NO). Therefore, the control unit 160 determines that the abnormality in administration occurs (S302). Next, the control unit 160 stops the DC motor 141 by blocking the current supplied to the DC motor 141 (S303), and notifies a user of the abnormality in administration (S304). A form of the notification of the abnormality in administration is the same as that in the first embodiment.

The liquid medicine administration device of the present invention has been described above through a plurality of the embodiments, but the present invention is not limited to each of the configurations described above, and can be appropriately changed based on the description of the claims.

### Reference Signs List

- 10: Liquid medicine administration system
- 100: Liquid medicine administration device
- 110: Liquid medicine container
- 111: Main body of liquid medicine container
- 111a: Lumen of liquid medicine container
- 112a: Distal end inner wall of liquid medicine container
- 120: Housing
- 120a: Housing main body
- 127: Chassis
- 128: Accommodation space
- 130: Plunger
- 131: Main body of plunger
- 134: Portion to be detected
- 135: Gasket
- 135a: Distal end of plunger
- 140: Drive mechanism
- 141: DC motor
- 143: Speed reduction mechanism
- 144: Photointerrupter
- 145: Slit plate
- 146: Encoder
- 148: Current limiting circuit
- 150: Detection unit
- 160: Control unit
- H: Body surface

## Claims

1. A liquid medicine administration device comprising:
a plunger that pushes a liquid medicine in a liquid medicine container filled with the liquid medicine;
a supply path that causes a distal end opening of the liquid medicine container to communicate with a needle in order to administer the liquid medicine from the needle pierced into a living body;
a drive mechanism that advances the plunger toward the distal end opening of the liquid medicine container in order to discharge the liquid medicine from the liquid medicine container; and
a control unit that controls operation of the drive mechanism, wherein
the drive mechanism includes a DC motor that applies, to the plunger, a drive force for advancing the plunger, and a rotation detection unit that detects a rotation of the DC motor, and
the control unit has a rotation speed calculation function of calculating a rotation speed of the DC motor based on the rotation of the DC motor detected by the rotation detection unit, and detects an abnormality in administration when the rotation speed of the DC motor calculated by the rotation speed calculation function is lower than a predetermined lower limit rotation speed.

2. The liquid medicine administration device according to claim 1, wherein the rotation detection unit is an encoder including a photointerrupter and a slit plate, the slit plate rotating in accordance with the rotation of the DC motor.

3. The liquid medicine administration device according to claim 1 or 2, wherein the control unit stops the DC motor when detecting the abnormality in administration.

4. The liquid medicine administration device according to claim 1 or 2, wherein when the abnormality in administration is detected, the control unit notifies that the abnormality in administration occurs.

5. A liquid medicine administration device comprising:
a plunger that pushes a liquid medicine in a liquid medicine container filled with the liquid medicine;
a supply path that causes a distal end opening of the liquid medicine container to communicate with a needle in order to administer the liquid medicine from the needle pierced into a living body;
a drive mechanism that advances the plunger toward the distal end opening of the liquid medicine container in order to discharge the liquid medicine from the liquid medicine container; and
a control unit that controls operation of the drive mechanism, wherein
the drive mechanism includes a DC motor that applies, to the plunger, a drive force for advancing the plunger, and
the control unit has a current value measurement function of measuring a value of a motor current flowing in the DC motor, and detects an abnormality in administration when the value of the motor current exceeds a predetermined upper limit current value.

6. A liquid medicine administration device comprising:
a plunger that pushes a liquid medicine in a liquid medicine container filled with the liquid medicine;
a supply path that causes a distal end opening of the liquid medicine container to communicate with a needle in order to administer the liquid medicine from the needle pierced into a living body;
a drive mechanism that advances the plunger toward the distal end opening of the liquid medicine container in order to discharge the liquid medicine from the liquid medicine container and includes a DC motor that applies, to the plunger, a drive force for advancing the plunger;
a control unit that controls operation of the drive mechanism; and
a current limiting circuit that limits a current so that any current equal to or greater than a predetermined current does not flow in the DC motor.
